# EUROPEAN PATENT APPLICATION

(11) **EP 0 753 308 A2**
(43) Date of publication of application: **15.01.1997**
(21) Application number: 96830375.0
(22) Date of filing: 03.07.1996
(51) Int. Cl.: A61K 38/40

(54) **Use of lactoferrin for therapy of acute or recurrent infectious diseases by streptococcus pyogenes or other intracellular gram positive pathogen bacteria**

(30) Priority: 12.07.1995 IT RM950473
(71) Applicant: Gambit International Limited, Tortola (VG)
(72) Inventor: Valenti, Piera, 00153 Rome (IT); Antonini, Giovanni, 00153 Rome (IT)

(57) **Abstract**

The present invention relates to the therapeutic utilization of the protein lactoferrin for the therapy of many acute or recurrent bacterial infectious diseases of men and animals. In details, the present invention demonstrates an anti-invasive property of lactoferrin in apo and iron-saturated form towards intracellular Gram positive pathogen microorganisms, like *Streptococcus pyogenes* and *Staphylococcus aureus* not being related to the well known antibacterial activity of lactoferrin and to the other known properties of the protein. The same anti-invasive activity of lactoferrin is also possessed, even if to a lower extent, by polypeptide fragments of the protein and by other transferrin proteins eventually chemically modified.

## Description

### TECHNICAL FIELD

The present invention relates to the therapeutic utilization of lactoferrin for preventing and curing many bacterial infectious diseases of men and animals, caused by intracellular Gram positive pathogen microorganisms.

### BACKGROUND ART

A first step of bacterial infection can be represented by the adhesion of bacteria to the host cell. Some pathogen bacteria are able to adhere to the cell through specific surface molecules while others are to invade the cell, to survive in the endosome or, escaping from the vacuole, to replicate within the cytoplasm. The molecules that allow bacteria to internalize into the cells are considered virulence factors because bacteria in intracellular phase, evade the host immune system and drugs. Intracellular bacteria can multiply in the cell, causing an acute infection, or can survive into the cell recuring in some circumstances. This last event can explain the recurrence of bacterial infections of ephitelium and mucosas. An example of this phenomenum is well represented by the recurrent infections of oropharynx caused by *Streptococcus pyogenes*. Group B-hemolytic Streptococci are responsible of several diseases such as erysipelas, puerperal fever, sepsis, streptococcical angina, impetigo, genital-urinary infections, rheumatic fever and nephritis. In particular, streptococcical angina, caused by Group A-Streptococci, is a recurrent infection very usual in children. These bacteria exert their pathogenicity by colonizing the pharynx epithelium adhering to host cells with specific molecules and producing in the human host rhinopharyngitis, tonsillitis, mucous edema, swelling and ache of cervical lymphonodes. Even after an antibiotic treatment (usually penicillin or erytromycin), it has been widely demonstrated the appearance of a lot of recurrent infectious diseases. In fact, following an appropriate antibiotic therapy owing to the patient full clinical recovery (this occurrence can be supported by a pharyngeal isolate negative for *Streptococcus pyogenes*) is possible to have an reinfection in 2-4 weeks.

The property of *S. pyogenes* to cause recurrent infection diseases can be now attribuite to the invasive capability, recently demonstrated, by which this bacterium can adhere and enter epithelial cells avoiding any effect either from the immune system of the host or from an antibiotic treatment. It has been also demonstrated the capability of *Staphylococcus aureus* to invade host epithelial cells. In fact, this pathogen bacterium colonizes human skin and causes suppurative infectious diseases. *Staphylococcus aureus* is also responsible of deep infections such as abscesses, osteomyelitis, clinical treatment pyelonephritis, penumonia, empyema, purulent arthritis, septicemia and endocarditis.

It is therefore possible to understand the importance of therapeutic treatment in order to preventing and inhibit the adhesion and the invasion of epithelial cells by *Streptococcus pyogenes* and other Gram positive invasive microorganisms. This therapy, hindering the bacterial internalization, will help the antibiotic treatment and, at the same time, will prevent the recurrence of infections. Up to now, there are not drugs on market that can be utilized for this purpose in humans and animals.

### DISCLOSURE OF THE INVENTION

The therapeutic model, described in the present invention, is based on the utilization of the anti-invasive activity of lactoferrin (or its analogous molecules or its fragments) towards bacteria. This anti-invasive property of lactoferrin represents a new approach for the therapy of infectious diseases caused by intracellular Gram positive pathogen bacteria. Lactoferrin topic treatment is characterized by very low or no toxic effects and, therefore, can be utilized in bacterial infections concerning skin and mucosas like oropharynx, nasal, intestinal, bronchial, vaginal or other mucosas. The therapeutic utilization of this anti-invasive property possessed by lactoferrin is not related to the antibacterial activity of the molecule and, in a more general way, the antiinvasive property, is possessed by proteins analogous to lactoferrin, generically called transferrins, and is also possesed by fragments of these proteins even to a lesser extent. Since at the present time there are no *in vivo* models for testing the anti-invasive properties of substances towards *S. pyogenes*, the examples below described have been performed to *in vitro* models that are generally accepted for assaying anti-invasive molecules. This anti-invasive activity possessed by lactoferrin (or its analogous molecules or its fragments) towards invasive bacteria is well demonstrated *in vitro* by the lawer number or absence of *S. pyogenes* inside cultured epithelial human HeLa cells, when lactoferrin at non cytoxicic and non bactericidal concentrations (e.g. up to 2mg/ml) is present at the same time with the cells and the microorganisms.

Furthermore, it has been demonstrated that lactoferrin possesses an anti-invasive activity towards other intracellular Gram-positive pathogen bacteria like *Staphylococcus* *aureus*, indicating a therapeutic use of lactoferrin in order to prevent and cure other acute or recurrent infections dependent on the internalization of a Gram positive pathogen microorganism in the host cells.

### WAYS OF CARRYING OUT THE INVENTION

Lactoferrin from bovine milk is the particulary preferred protein according to the invention. The examples, below reported, show that bovine lactoferrin can be considered optimum, with respect to treatments currently in use, for the prevention and cure of recurrent infectious diseases from intracellular Gram positive pathogen microorganisms. Furthermore, the extremely low toxicity of said protein contained in the formula according to the present invention is well-known, since it is a 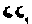 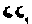 natural 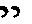 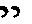 substance extracted from bovine milk and is very similar, in its amino acid composition and structural configuration, to the homologous protein that is normally present in humans. This is an additional advantage of bovine lactoferrin with respect to the chemicals currently used in infection treatments. The protein that is used according to the present invention can be extract or can be obtained by recombinant-DNA technology or also by chemical synthesis.

The term lactoferrin designates a glycoprotein present in colostrum and milk, in many biological secretions and in the leucocyte granules of mammalians. Lactoferrin possesses an isoelectric point of 7.8, a molecular weight of about 83,000 Da, two sugar chains and a variable saturation with iron. Each molecule of lactoferrin binds 2 atoms of Fe³⁺ in the presence of sodium bicarbonate. The main function of this protein is to reduce the amount of free iron in biological liquids, inhibiting the bacterial growth (the bacteriostatic property of lactoferrin is already well known) and decreasing the risk of forming free radicals due to the presence of not chelated iron. Lactoferrin belongs to a family of glycoprotein generally called "Transferrin", characterized in so that they possess two binding sites for Fe³⁺ per molecule and a high degree of homology among them. Bovine lactoferrin can be industrially produced by purification from bovine milk.

The therapeutic utilization of the anti-invasive activity of lactoferrin towards intracellular Gram-positive pathogen bacteria is the main claim of this disclosure while it is not relevant, as regard with the anti-invasive activity, the source of the lactoferrin (i.e. from extractive chemistry or from recombinant DNA technique).

The anti-invasive activity described in the present disclosure, is possessed by all the marketable proteins known as lactoferrin such as human lactoferrin or mouse lactoferrin and by other proteins belonging to the 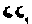 Transferrin family 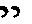 (even if they show a lesser anti-invasive activity) produced by extraction or utilizing recombinant DNA technique, and, therefore, the anti-invasive activity of lactoferrin has to be extended, as described in the present disclosure, to all the lactoferrins, from various sources, to transferrins and related proteins, from various natural or industrial sources.

The anti-invasive activity of lactoferrin has been showed at not toxic concentrations of lactoferrin towards human cultured cells and at non antibactericial concentrations towards the tested microoganisms; all the preparation of lactoferrin with different iron saturation from apo-form to totally Fe³⁺ saturated form, showed the same antiinvasive property. The antiinvasive activity of lactoferrin towards microorganisms, whose therapeutic utilization is described in the present disclosure, is also possessed by its fragments, obtained by chemical and enzimatic treatments or obtained through chemical synthesis or other technologies like recombinant DNA technique.

The lactoferrin activity according to the present invention can be obtained using lactoferrin stored in liquid form, as solution at concentration of 0.1 to 10%, e.g. 1 to 5%, weight/volume (g/ml) of the protein in solvents accettable for pharmaceutial use, in particular water or hydro-alcoholic solvents such as water-ethanol mixtures, or in solid form (lyophilized, dried, frozen) and in the other commonly known forms of storage: for example immobilized or adsorbed on an inert support commonly used in the pharmaceutical field.

Lactoferrin, with respect to the activity according to the invention, can thus be used in the liquid form, as a rinse or gargle, or in solid form, such as granular form to be dissolved in water for preparing a gargle just before use, with the concentrations specified above or also in powder for use on skin. Alternatively, it is possible to incorporate the protein mixture of the invention in a formulation to be chewed, such as chewing gum, tablets, pastilles, lozenges, etc. in a concentration of 1 to 60% by weight of the total formulation.

In its form ready-for-use, the lactoferrin formulation with respect to the activity according to the invention, can also comprise further conventional antibiotic and antibacterial compounds as well as carries, fillers, flavouring agents, preservatives, surfactants, colorants and other adjuvants selected from those conventionally used for the various liquid or solid form preparations for oral topical use and for skin topical use.

Thus, lactoferrin formulation, with respect to the activity according to the invention, can also comprise antibiotic compounds like: penicillins, cephalosporins, chloromphenicol, macrolids, aminoglicosids, sulphamidics etc. and further anti-bacterial compounds, such as quaternary ammonium compounds with one long chain alkyl on the nitrogen atom, alkali metal pyrophosphates and orthophosphates, halogenated bisphenols and halogenated diphenyl ethers, sodium benzoate, sodium salicylate, etc.

Lactoferrin formulation, with respect to the activity according to the invention, can further comprise humectants, e.g. glycerin, sorbitol, xylitol, propylene glycol, atc., flavours, e.g. oil of spearmint; peppermint or cinnamon, menthol, methyl salicylate, atc., sweetening agents, e.g. aspartame, saccharin, dextrose, cyclamate, wintergreen, etc., thickening agents, e.g. xantan gum, carrageenin, carboxy methyl cellulose, etc..

The formulation to be chewed by the user will comprise the respective conventional base material and conventional adjuvant such as flavouring, sweetening and coloring agents, humectants, atc., as those mentioned above; and thickening and gelling agents such as thickening silica, natural or synthetic gums, e.g. tragacanth gum, guar gum, hydroxyethyl- and, carboxymethyl cellulose, polyvinyl pyrrolidone, starch, etc..

Lactoferrin formulation, with respected to the activity according to the invention, in either liquid or solid form, should be used for purposes of prevention of recurrent infectious diseases by *S.pyogenes* or by *S.aureus* at last once, preferably twice a day; for purposes of treatment the frequency of use can be increased to 3 to 4 times a day.

The following examples give a strong evidence of the therapeutical utilization of antiinvasive activity of lactoferrin (or its analogous molecules or its fragments) towards intracellular Gram positive pathogen microorganisms. This utilization, described in the present disclosure, can be considered optimal as regards with the common therapeutical treatments, because of its utilization alone or as adjuvant in antibacterial therapies concerning acute or recurrent infections by intracellular Gram positive pathogen bacteria.

### EXAMPLES

The evaluation of the invasive capability of *S. pyogenes* must occur through a specific experimental procedure that we described below:

### Bacterial strains and cultures

*Streptococcus pyogenes* strain 95/1 was cultured in Todd-Hewitt broth (Difco lab.). Blood agar was used for counting coloning forming units (C.F.U.).
*Staphylococcus aureus* strain 94/3 was cultured in Brain Hearth Infusion (BHI). Mannitol Salt Agar was used for C.F.U. counts.
HeLa S3 human epitelial cells (derived from epithelioid carcinoma of human cervix) were grown as monolayers at 37°C in Eagle's MEM containing 1.2 g/l of NaCo_{3,} 2 mM glutamine, 100 U/ml penicillin, 0.1 mg/ml streptomycin and 10% heat inactivated foetal calf serum in 5% CO₂ on 24-well tissue culture clusters (costar) by loading 1 ml of a solution of 1.5x10⁵ cells/ml.

### Invasion assay and test for anti-invasive activity of lactoferrin

Semiconfluent monolayers of HeLa cells were infected with a bacterial suspension (at a multiplicity of infection of 100 exponentially grown bacteria per cell) deriving from a bacterial subculture after an incubation at 37°C for 120 min in the presence and absence of lactoferrin added at no cytotoxic nor antibacterial concentrations. After the bacterial infection (2 h at 37°C ), the monolayer was five-fold carefully washed with fosfate buffered saline (PBS) and then fresh medium (MEM, Seromed) containing 200 ug/ml of gentamicin was added. This antibiotic is well known for its impossibility to penetrate into the eukaryotic cells and to exert its bactericidal effect only towards the extracellular bacteria (free or adhered to the cells). Alter 2 h incubation period at 37°C in gentamicin, the infected cells were whashed three times with PBS and lysed by the addition of cold 0.1% Triton-X 100.

The cellular lysate was diluited with PBS and plated on selective agar medium in order to determine viable intracellular bacteria.

### EXAMPLE 1

### Anti-invasive activity of lactoferrin towards S. pyogenes 95/1 at non cytotoxic and non bacterial concentrations.

First of all, we evaluated the minimal toxic concentration towards the cells. Different lactoferrin concentrations were kept in contact with cellular monolayers. After 24 h incubation period, the monolayers were examined by optical microscopy after vital staining.

**Table 1**

| **Toxicity of different concentrations of lactoferrin towards HeLa cells.** | |
|---|---|
| **Lactoferrin (mg/ml)** | **HeLa cells** |
| 0 | - |
| 0,25 | - |
| 0.50 | - |
| 1.00 | - |
| 2.00 | - |
| 4.00 | - |
| 8.00 | - |
| 50.00 | + |
| **The cytotoxic effect was evaluated by examining the cell morphology and vitality** **(-) no cytotoxic effect** **(**+**)** presence **of cytotoxicity** | |

The lactoferrin was also tested in order to quantify the minimal concentration not inhibiting the bacterial growth.

**Table 2**

| **Antibacterial activity of lactoferrin towards *S. pyogenes* 95/1.** | |
|---|---|
| **Lactoferrin (mg/ml)** | ***S.pyogenes*** **(CFU/ml)** |
| 0 | 2 x 10⁸ |
| 0.5 | 3 x 10⁸ |
| 1.0 | 2 x 10⁸ |
| 2.0 | 2 x 10⁸ |
| 5.0 | 5 x 10⁷ |
| 10.0 | 1 x 10⁷ |

The antibacterial activity of lactoferrin was carried out in Todd Hewith broth incubated at 37°C for 18 h. After this period, the aliquots of the mixture were plated on Blood agar and the viable bacteria were quantified by CFU counts. The inoculum consisted of about 5 x 10⁵ cell/ml.
The anti-invasive effect of lactoferrin at non cytotoxic and non bactericidal concentration (1 mg/ml) was tested utilizing *S. pyogenes* 95/1 added to HeLa cell monolayers.

**Table 3**

| **Effect of lactoferrin on the invasion ability of** ***S. pyogenes*** **95/1** | |
|---|---|
| **Lactoferrin (mg/ml)** | **Internalized bacteria (CFU/ml)** |
| 0 | 5 x 10⁵ |
| 1.0 | 5 x 10³ |
| 2.0 | 1 x 10³ |

The anti-invasive effect of lactoferrin is demonstrated by a 100/500-fold decrease of the number of internalized bacteria.

The anti-invasive effect of lactoferrin has been also compared with that obtained utilizing ovotransferrin, a protein analogous to lactoferrin, at non cytotoxic and non bacterialidal concentrations. The data are showed in Table 4.

**Table 4**

| **Anti-invasive activity of ovotransferrin in comparison with lactoferrin towards** ***S. pyogenes*** **95/1** | | |
|---|---|---|
| **Lactoferrin (mg/ml)** | **Ovotransferrin (mg/ml)** | **Internalized bacteria CFU/ml)** |
| 0 | 0 | 5 x 10⁵ |
| 1.0 | 0 | 5 x 10³ |
| 0 | 1.0 | 1 x 10⁵ |

The Table 4 shows that the anti-invasive effect of ovotransferrin is much lesser than that observed with lactoferrin. All the molecules, generally called transferrins, possess, even if in a different degree, an anti-invasive activity towards *S. pyogenes* 95/1.
It must be underlined that the above mentioned anti-invasive activity of lactoferrin and other transferrins is not affected by the degree of iron saturation of these proteins.

### EXAMPLE 2

### Anti-invasive activity of lactoferrin towards S. pyogenes 95/1 in presence of subinhibiting concentrations of antibiotics.

It is well known as *S. pyogenes* 95/1 is sensible to the antibacterial action of erythromycin and ampicillin. Subinhibiting amounts of erythromycin or ampicillin were utilized in order to verify if the bacteria pretreated with this antibiotic are inhibited in their invasion efficiency.
For this reason, *S. pyogenes* 95/1 was cultured in presence of subinhiting concentrations of antibiotics and the invasivity test was performed with this bacterial inoculum. The data are reported in Table 5.

**Table 5**

| **Effect of subinhibiting concentrations of antibiotics towards the invasivity of** ***S. pyogenes*** **95/1 in absence of lactoferrin.** | |
|---|---|
| **Antibiotics (ug/ml)** | **Internalized bacteria (CFU/ml)** |
| 0 | 5 x 10⁵ |
| Erythromicin 20 | 2 x 10⁵ |
| Ampicillin 0.1 | 2 x 10⁵ |

The reported data demonstrate that bacteria pretreated with antibiotics do not lose their invasion capability.
It was tested, therefore, the anti-invasive activity of lactoferrin towards *S. pyogenes* 95/1 pretreated with subinhibiting amounts of erythromycin or ampicillin. The effect of lactoferrin was tested towards *S. pyogenes* 95/1 grown for 24 h in presence of erythromycin (20 ug/ml) or in presence of ampicillin (0.1 ug/ml). The results are showed in Table 6.

**Table 6**

| **Anti-invasive activity of lactoferrin towards** ***S. pyogenes*** **95/1 pretreated with antibiotics.** | | |
|---|---|---|
| **Lactoferrin (mg/ml)** | **Antibiotic (ug/ml)** | **Intracellular bacteria (CFU/ml)** |
| 0 | 0 | 5 x 10⁵ |
| 1 | 0 | 6 x 10³ |
| 2 | 0 | 4 x 10³ |
| 1 | Erythromicin 20 | 3 x 10 |
| 2 | Erythromicin 20 | 0 |
| 1 | Ampicillin 0.1 | 0 |

The same test was performed with other antibiotics and fairly similar results were obtained. It is well evident that the anti-invasive activity of lactoferrin increases when bacteria are pretreated with antibiotics, demonstrating the co-operative effect of a therapeutic treatment of lactoferrin and antibiotics against infectious diseases by intracellular pathogen bacteria.

### EXAMPLE 3

### Anti-invasive activity towards S. pyogenes 95/1 possessed by lactoferrin and ovotransferrin chemically or enzimatically modified.

The performed modifications are described below:
- the fragment 1 was obtained by treating the lactoferrin with a mixture of proteolytic enzimes without any further purification. The electrophoresis analysis showed the presence of different fragments with a molecular weight lesser than the untreatred lactoferrin and the absence of intact lactoferrin;
- the fragment 2 was obtained by treating the lactoferrin with a mixture of enzymes endoglycosidases and esoglycosidases without any further purification. The analysis of glucides showed a decrease of about 70% of the glycosilation degree of lactoferrin.
- the glycosilate ovotransferrin was obtained by incubating the protein at alkaline pH with high glucide concentrations, according with the common methods to increase the glycosilation of proteins.

**Table 7**

| **Anti-invasive activity towards** ***S.pyogenes*** **95/1 possessed by modified transferrins utilized at the concentration of 1 mg/ml.** | |
|---|---|
| **Protein** | **Intracellular bacteria (CFU/ml)** |
| None (control) | 5 x 10⁵ |
| Lactoferrin | 5 x 10³ |
| Ovotransferrin | 1 x 10⁵ |
| Fragment 1 | 5 x 10⁴ |
| Fragment 2 | 8 x 10⁴ |
| Glycosilate ovotransferrin | 1 x 10⁴ |

From the above Table is evident that all the modified preparation show an anti- invasive activity.

### EXAMPLE 4

### Protective activity of lactoferrin towards the cell penetration of other intracellular pathogen Gram positive bacteria.

The anti-invasive activity of lactoferrin towards other intracellular pathogen Gram positive bacteria has been showed by means of the described protocol.

**Table 8**

| **Anti-invasive activity of lactoferrin, its analogous and fragments (at the concentration of 1 mg/ml) also in presence of subinhibiting amounts of erythromycin (0.02 mg/ml) towards intracellular pathogen Gram positive bacteria.** | | |
|---|---|---|
| **Proteins** | **Intracellular bacteria (CFU/ml** | |
| | ***S. pyogenes*** | ***S. aureus*** |
| None (Control) | 5 x 10⁵ | 7 x 10⁵ |
| Erythromycin | 2 x 10⁵ | 3 x 10⁵ |
| Apo-lactoferrin | 5 x 10³ | 6 x 10³ |
| Iron saturated lactoferrin | 5 x 10³ | 6 x 10³ |
| Fragment 1 | 5 x 10⁴ | 4 x 10⁵ |
| Fragment 2 | 8 x 10⁴ | 3 x 10⁵ |
| Glycosilate Ovotransferrin | 1 x 10⁴ | 1 x 10⁴ |
| Lactoferrin + Erythromycin | 0 | 0 |
| Lactoferrin + Ampicillin | 0 | 0 |
| Fragment 1+ Erythromycin | 2 x 10² | 2 x 10³ |
| Fragment 1 + Ampicillin | 1 x 10² | 3 x 10³ |
| Fragment 2 + Erythromycin | 3 x 10² | 3 x 10³ |
| Fragment 2 + Ampicillin | 1 x 10² | 2 x 10³ |
| Glycosilate Ovotransferrin + Erythromicin | 1 x 10 | 8 x 10 |
| Glycosilate Ovotransferrin + Ampicillin | 2 x 10 | 6 x 10 |

The experiments reported in Table 8 demonstrate that the lactoferrin and its analogous, such as ovotransferrin and glycosilate ovotransferrin and its fragments such as the fragment 1 above described, are active towards all the examined intracellular Gram positive pathogen bacteria and that this activity is increased in presence of subinhibiting amounts of antibiotic molecules.

### EXAMPLE 5 -Gargle

Composition per 100 ml

### Active ingredients :

Lactoferrin, from bovine milk, SIGMA Chemical Co, cat L 4765, 1.0 g.

### Carriers, preservatives and flavouring agents:

Sodium chloride 1 g
Sodium bicarbonate 100 mg
Methyl-p-hydroxybenzoate 100 mg
Peppermint oil 50 mg
Purified water to 100 ml

### Use:

For prevention and curing Streptococcical angina two gargle a day, one in the morning, one in the evening.

### EXAMPLE 6 - Envelope packs

Composition for 1 envelope

### Active ingredients:

Lactoferrin, from bovine , SIGMA Chemical Co., cat L4765, 1.0 g.
Erytromicin 0.2 g

### Carriers, preservatives and flavouring agents:

Sodium choloride 20 mg
Sodium bicarbonate 10 mg
Methyl-p-hydroxybenzoate 20 mg
Peppermint oil 5 mg

### Use:

For preventing and curing Streptococcical angina, the content of an envelope is dissolved in 20 ml water for two rinse, one in the morning and one in the evening.

### EXAMPLE 7 - Chewing gum

Composition of one piece of gum :

### Active ingredients:

Lactoferrin, from bovine milk, SIGMA Chemical Co. cat L4765, 250 mg.
Benzalkonium chloride 10 mg

### Carries, preservatives and flavouring agents:

Gum base (Paloya TX) 400 mg
Glucose 100 mg
Glycerol 10 mg
Sodium bicarbonate 10 mg
Methyl-p-hydroxybenzoate 20 mg
Peppermint oil 5 mg

### Use:

For preventing and curing Streptococcical angina, one piece of chewing gum in the morning, one in the evening.

### EXAMPLE 8 - Powder

Composition for 100 g

### Active ingredients:

Lactoferrin from bovine milk, SIGMA Chemical Co., cat L 4765, 10 g
ampicillin 1g

### Carriers, preservatives

corn starch 85 g
zin oxide 3 g
thickening silica powder 1 g

### Use:

For curing staphilococcical infections of skin apply twice a day.

## Claims

1. Therapeutic utilization of lactoferrin for preventing or treating infectious diseases by intracellular Gram positive pathogen bacteria. Lactoferrin for therapeutic utilizzation in preventing and treating infectious diseases by intracellular Gram positive pathogen bacteria characterized in that said protein inhibits invasion of said microorganisms into host cells.

2. Therapeutic utilization of the anti-invasive activity of lactoferrin, according to claim 1, utilized for preventing or treating infectious diseases by *Streptococcus pyogenes* concerning oropharynx of infants and adults and for preventing and treating related recurrent infectious diseases in humans.

3. Therapeutic utilization of the anti-invasive activity of lactoferrin according to claim 1, towards other intracellular Gram positive pathogen bacteria such as *Staphylococcus aureus*, for preventing or treating acute and recurrent bacterial infections concerning skin and different type of mucosas such as oropharynx, nasal, intestinal, bronchial, vaginal or other mucosas.

4. Therapeutic utilization of the anti-invasive activity of lactoferrin towards *Streptococcus pyogenes* and other intracellular Gram positive pathogen bacteria, according to claims 1,2 and 3, possessed by lactoferrin with a variable saturation in Fe³⁺.

5. Therapeutic utilization of the anti-invasive activity of lactoferrin towards *Streptococcus pyogenes* and other intracellular pathogen Gram positive bacteria, according to claims 1,2 and 3, possessed by lactoferrin or by proteins or peptides characterized by high sequence omology with lactoferrin like native or chemically modified transferrins, extracted by natural sources: animals or vegetables, or produced by chemical synthesis or by other technology such as recombinant DNA tecniques.

6. Therapeutic utilizzation of the antiinvasive activity of lactoferrin, according to claims 1,2,3,4 and 5, when said protein is in liquid preparation, solid preparation and preparation immobilized or adsorbed on an inert support.

7. Therapeutic utilizzation of the antiinvasive activity of lactoferrin, according to claims 1,2,3,4 and 5, when said protein is in liquid preparation, solid preparation and preparation immobilized or adsorbed on an inert support, in a gargle formulation, envelope packs, chewing gum or powder in a 0,1 to 10% by weight.

8. Therapeutic utilizzation of the antiinvasive activity of lactoferrin, according to claims 1,2,3,4 and 5, when the protein is in liquid prearation, solid preparation and preparation immobilized or adsorbed on inert support, associated to one or more antibiotics or antibacterial agents.

9. Therapeutic utilizzation of the antiinvasive activity of lactoferrin, according to claims 1,2,3,4 and 5, the protein being in liquid prearation, solid preparation and in preparation immobilized or adsorbed on inert support, associated to one or more adjuvants selected from humectants, sweeteners, flavouring agents, thckening agents acceptable for topical use on skin or mucosas.

10. Methods of using lactoferrin, according to claims 1,2 and 3 for preventing or treating bacterial infections comprising topically administering said protein to the mucosas or skin at least once a day.
